# EUROPÄISCHE PATENTANMELDUNG

(11) **EP 3 741 396 A1**
(43) Veröffentlichungstag der Anmeldung: **25.11.2020**
(21) Anmeldenummer: 20175789.5
(22) Anmeldetag: 20.05.2020
(51) Int. Cl.: A61L 9/12, A47K 10/42, B65D 83/08, B60H 3/00, B60R 7/08

(54) **MEHRZWECKBEHÄLTER ZUM BEREITSTELLEN VON TÜCHERN MIT EINER LUFTERFRISCHUNGSFUNKTION**

(30) Priorität: 22.05.2019 DE 102019113672
(71) Anmelder: Cupssy GmbH, 61137 Schöneck (DE)
(72) Erfinder: Venturino, Enzo, 61137 Schöneck (DE)
(74) Vertreter: Patentanwälte Olbricht Buchhold Keulertz

(57) **Zusammenfassung**

Die Erfindung betrifft einen Mehrzweckbehälter (1) mit einer Lufterfrischungsfunktion zum Aufnehmen von Tüchern (31), insbesondere für den Einsatz in einem Getränkehalter eines Fahrzeugs. Der Mehrzweckbehälter umfasst ein Gehäuse (10) mit einem Deckel (20) zum Ausbilden eines Aufnahmeraums (13) in dem Gehäuse (10), in den eine Kartusche (30) mit z.B. Reinigungstüchern eingesetzt werden kann.

Der Deckel (20) weist eine Öffnung (22) zum Entnehmen von Tüchern (31) aus dem Aufnahmeraum (13) und eine Aufnahmevertiefung (21) zum Aufnehmen eines Dufteinsatzes (40) auf.

## Beschreibung

Die Erfindung betrifft einen Mehrzweckbehälter mit einer Lufterfrischungsfunktion und zum Bereitstellen von Tüchern, insbesondere für den Einsatz in einem Getränkehalter eines Fahrzeugs gemäß dem Oberbegriff von Anspruch 1. Des Weiteren betrifft die Erfindung einen Dufteinsatz sowie eine Kartusche.

Eine Vielzahl von Fahrzeugen ist derzeit mit Getränkehaltern bzw. Becherhaltern ausgerüstet. Beispielsweise können derartige Getränkehalter in einer Mittelkonsole, an einem Handschuhfach oder im Bereich von Rücksitzen des Fahrzeugs angeordnet sein. Insbesondere in der Mittelkonsole angeordnete Getränkehalter sind üblicherweise doppelt ausgeführt.

DE 10 2005 028 586 A1 beschreibt beispielsweise einen Getränkehalter zum Aufnehmen von Bechern oder Getränkeflaschen. Der Getränkehalter ist in eine Mittelkonsole des Fahrzeugs integriert und weist zwei Aufnahmeplätze für Becher oder Flaschen auf. Oftmals wird nur ein Aufnahmeplatz, beispielsweise für das Ablegen von Getränkeflaschen, verwendet. Der zweite Aufnahmeplatz bleibt bei derartigen Getränkehaltern häufig ungenutzt.

Der Erfindung liegt nun die Aufgabe zugrunde, einen Mehrzweckbehälter vorzuschlagen, welcher ungenutzte Aufnahmeplätze von Getränkehaltern mit zusätzlichen Funktionen ausstattet.

Hauptmerkmale der Erfindung sind im kennzeichnenden Teil von Anspruch 1 sowie im Anspruch 12 und 14 angegeben. Ausgestaltungen sind Gegenstand der Ansprüche 2 bis 11 und 13.

Die Erfindung betrifft einen Mehrzweckbehälter mit einer Lufterfrischungsfunktion zum Bereitstellen von Tüchern, insbesondere für den Einsatz in einem Getränkehalter eines Fahrzeugs. Der Mehrzweckbehälter weist ein Gehäuse und einen Deckel zum Verschließen des Gehäuses und zum Ausbilden eines Aufnahmeraums in dem Gehäuse auf. Desweiteren weist der Mehrzweckbehälter einen Dufteinsatz auf. Erfindungsgemäß weist der Deckel eine Öffnung zum Entnehmen von Tüchern aus dem Aufnahmeraum auf, wobei im Deckel eine Aufnahmevertiefung zum Aufnehmen des Dufteinsatzes vorgesehen ist.

Der Mehrzweckbehälter kann vorzugsweise als ein durch den Deckel verschließbarer Becher ausgestaltet sein, welcher in einen Aufnahmeplatz eines Getränkehalters einsetzbar ist. Hierzu kann das Gehäuse eine im Wesentlichen zylindrische Form aufweisen, welche in Richtung des Deckels aufgeweitet ist. Hierdurch kann der Mehrzweckbehälter auch bei klappbaren sowie bodenlosen Getränkehaltern verwendbar sein.

Des Weiteren kann der Mehrzweckbehälter eine bodenseitige Adapterhülse aufweisen, durch welche der Mehrzweckbehälter an Getränkehalter mit größeren Durchmessern einstellbar ist. Die Adapterhülse kann als ein Überzug aus einem elastischen Material, wie beispielsweise Silikon oder Elastomer, ausgeführt sein.

Der auf das Gehäuse angebrachte Deckel begrenzt ein Innenvolumen des Gehäuses und bildet somit einen Aufnahmeraum aus. Der Aufnahmeraum dient vorzugsweise zum Aufnehmen von Tüchern. Über die in den Deckel eingebrachte Öffnung können die Tücher einzeln entnommen werden.

In einer vorteilhaften Ausgestaltung kann nach einem Entnehmen eines Tuches ein Ansatz eines nachfolgenden Tuches in der Öffnung des Deckels verbleiben, sodass ein Entnehmen von Tüchern aus dem Aufnahmeraum vereinfacht wird.

Die Tücher können beispielsweise als Papiertaschentücher, Reinigungstücher, Feuchttücher, Glasreinigungstücher für Fensterscheiben oder Brillen, Lederpflegetücher, Tücher zur Kunststoffreinigung, Desinfektionstücher, Erfrischungstücher, Tücher zur Motorölstandskontrolle, parfümierte Tücher und dergleichen ausgestaltet sein. Somit sind verschiedene Anwendungsbereiche des Mehrzweckbehälters in Abhängigkeit von Tränkung und Art der Tücher möglich.

Darüber hinaus ist eine Änderung des Anwendungsbereichs des Mehrzweckbehälters durch einen Erwerb und einen Einsatz von abweichenden Tücher-Nachfüllpackungen möglich.

Durch die Verwendung eines Dufteinsatzes kann ein Funktionsumfang des Mehrzweckbehälters um eine Lufterfrischungsfunktion erweitert werden. Der Dufteinsatz kann vorzugsweise in unterschiedlichen Duftarten verfügbar und in der Aufnahmevertiefung des Deckels hineinsetzbar sein. Bevorzugterweise kann der Dufteinsatz als ein Duftkissen bzw. ein Duftpad ausgeführt sein, welcher neben der Öffnung des Deckels anbringbar ist. Der Dufteinsatz kann zum Freisetzen von Duftstoffen chemisch mit Luft reagieren können. Vorzugsweise kann der Dufteinsatz ringförmig geformt sein und konzentrisch zur Öffnung positionierbar sein. Somit bleiben die Tücher im Aufnahmeraum durch die Öffnung des Deckels weiterhin zugängig.

Der erfindungsgemäße Mehrzweckbehälter kann von Verbrauchern dazu verwendet werden, die nicht genutzten Getränkehalter bzw. Aufnahmeplätze von Getränkehaltern eines Fahrzeugs mit einer oder mehreren auf seine Bedürfnisse und Wünsche zugeschnittenen Erweiterungen in Form von Mehrzweckbehältern auszustatten und diese kostengünstig durch Zukauf von Dufteinsätzen und Tüchern über einen langen Zeitraum zu nutzen.

Gemäß einer Ausführungsform ist der Aufnahmeraum des Gehäuses dazu eingerichtet, Tücher aufzunehmen, wobei die Tücher in einer Kartusche oder direkt in dem Aufnahmeraum einsetzbar sind. Die Tücher können direkt und ohne zusätzliche Mittel in dem Gehäuse positionierbar sein. Vorzugsweise können die Tücher auf eine Form des Aufnahmeraums vorgefaltet bzw. geformt sein.

Alternativ können die Tücher in einer Kartusche bzw. einer Patrone angeordnet sein, wobei die Kartusche formschlüssig in den Aufnahmeraum hineinsetzbar ist. Durch den Einsatz einer Kartusche mit Tüchern kann ein Nachfüllen des Mehrzweckbehälters mit neuen Tüchern vereinfacht und beschleunigt werden. Die Kartusche kann vorzugsweise eine dem Aufnahmeraum entsprechende Form aufweisen. Insbesondere kann die Kartusche eine zylindrische Form aufweisen.

Die Handhabung des Dufteinsatzes kann vereinfacht werden, wenn der Dufteinsatz in eine Aufnahmeeinheit einlegbar ist, wobei die Aufnahmeeinheit mit dem eingelegten Dufteinsatz in der Aufnahmevertiefung des Deckels platzierbar ist. Beispielsweise kann die Aufnahmeeinheit als Bestandteil einer Verpackung des Dufteinsatzes ausgeführt sein, wobei der Dufteinsatz zwischen der Aufnahmeeinheit und einem abziehbaren Verpackungsdeckel verpackbar ist. Der Dufteinsatz kann mit der Verpackung in die Aufnahmevertiefung eingesetzt und der Verpackungsdeckel anschließend entfernt werden. Alternativ kann die Aufnahmeeinheit aus einem elastischen Material bestehen und für die formschlüssige Bevorratung des Dufteinsatzes vorgesehen sein.

Die Aufnahmeeinheit ist bevorzugterweise derart gestaltet, dass der Dufteinsatz in einer Dickenrichtung etwa 50% übersteht. Die andere Hälfte des Dufteinsatzes ist somit von der Aufnahmeeinheit abgedeckt. Dadurch wird eine lange Nutzungsdauer des Dufteinsatzes erreicht, da nur der überstehende Bereich Duftstoffe abgibt, während der durch die Aufnahmeeinheit abgedeckte Bereich nicht reagiert. Durch Drehen des Dufteinsatzes kann so eine Verdoppelung der Nutzungsdauer erreicht werden.

Nach einer weiteren Ausführungsform weist der Mehrzweckbehälter eine Dosiereinheit auf, welche den Dufteinsatz zumindest bereichsweise verdeckt. Die Dosiereinheit kann ist vorzugsweise relativ zum Dufteinsatz verschiebbar oder drehbar ausgestaltet. Die Dosiereinheit kann beispielsweise auf dem Dufteinsatz einseitig aufliegen und eine definierte Fläche des Dufteinsatzes abdecken. Hierdurch wird eine chemische Reaktion des Dufteinsatzes im Bereich der abgedeckten Fläche verhindert. Durch ein Verschieben oder Verdrehen der Dosiereinheit kann die abgedeckte Fläche bei Bedarf einer chemischen Reaktion mit Luft und somit einer Duftfreisetzung zugängig gemacht werden. Durch die Dosiereinheit kann somit die Intensität der Lufterfrischung am Deckel reguliert bzw. eingestellt werden. Des Weiteren kann die Verwendungsdauer des Dufteinsatzes durch die Dosiereinheit verlängert werden.

Gemäß einer weiteren Ausgestaltung ist die Dosiereinheit in die Aufnahmeeinheit oder in die Aufnahmevertiefung des Deckels über dem Dufteinsatz drehbar positionierbar. Die Dosiereinheit kann vorzugsweise eine runde Scheibenform aufweisen, welche mit einer Form der Aufnahmevertiefung korrespondiert. Hierdurch kann die Aufnahmevertiefung umfangsseitig als Drehlager für die Dosiereinheit dienen. Des Weiteren kann die Dosiereinheit als ein Verschluss der Aufnahmevertiefung ausgeführt sein, durch welchen der Dufteinsatz ortsfest am Deckel befestigt ist.

Die Tücher im Aufnahmeraum des Gehäuses können vor einem Austrocknen und einem Staubeintrag geschützt werden, wenn der Mehrzweckbehälter einen Verschluss zum Verschließen der Öffnung aufweist. Der Verschluss kann vorzugsweise als ein Stopfen ausgeführt sein, welcher die Öffnung luftdicht verschließen kann. Insbesondere kann der Verschluss gegen einen Verlust geschützt über eine flexible Halterung am Deckel befestigbar sein.

Nach einem weiteren Ausführungsbeispiel sind die in den Aufnahmeraum einsetzbaren Tücher als Feuchttücher ausgestaltet. Vorteilhafterweise weist das Gehäuse, der Deckel und/oder der Verschluss einen Feuchtigkeitssensor und/oder eine Feuchtigkeitsanzeige zum Anzeigen eines Feuchtigkeitsgehalts der Tücher im Aufnahmeraum auf. Hierdurch kann eine Anzeige des Feuchtigkeitsniveaus der Feuchttücher realisiert werden. Beispielsweise kann eine Feuchtigkeitsanzeige über ein Hygrometer oder über einen chemischen Indikator erfolgen, welcher im Verschluss oder im Deckel angeordnet ist.

Die Intensität des Dufteinsatzes kann besonders präzise und einfach eingestellt werden, wenn die Dosiereinheit einstellbare Luftschlitze zum Herstellen oder zum Verändern eines Luftkontakts zum Dufteinsatz aufweist. Hierzu kann die Dosiereinheit zwei oder mehr gegeneinander verdrehbare Abschnitte aufweisen, welche in einer Verschlussposition nebeneinander angeordnet sind und den Dufteinsatz vollständig verdecken. In einer geöffneten Position sind können sich die Abschnitte überlagern und somit einen Teil des Dufteinsatzes offenlegen.

Der Aufnahmeraum des Gehäuses kann technisch besonders einfach ausgebildet werden, wenn der Deckel über eine Schraubverbindung oder eine Steckverbindung mit dem Gehäuse verbindbar ist. Darüber hinaus kann somit ein unbeabsichtigtes Lösen des Deckels vom Gehäuse unterbunden werden.

Gemäß einer weiteren Ausführungsform ist die Öffnung zum Aufnahmeraum zentriert ausgerichtet ist und erstreckt sich durch den Deckel, die Aufnahmeeinheit, den Dufteinsatz und durch die Dosiereinheit. Hierdurch können alle Komponenten des Mehrzweckbehälters rotationssymmetrisch ausgebildet und konzentrisch zueinander ausgerichtet sein. Die Öffnung kann durch eine Rotationsachse der Komponenten des Mehrzweckbehälters verlaufen, wodurch der Zugang zu den Tüchern im Aufnahmeraum trotzt der Lufterfrischungsfunktion erhalten bleibt.

Eine besonders einfache Handhabung des Dufteinsatzes kann erreicht werden, wenn die Dosiereinheit und/oder die Aufnahmeeinheit und/oder der Dufteinsatz über Befestigungsclips oder über Rastverbindung ortsfest in der Aufnahmevertiefung des Deckels platzierbar sind. Somit kann eine formschlüssige Befestigung des Dufteinsatzes direkt oder mittels der Dosiereinheit in der Aufnahmevertiefung des Deckels realisiert werden.

Gemäß einem weiteren Aspekt der Erfindung wird ein Dufteinsatz bereitgestellt, wobei der Dufteinsatz in einem erfindungsgemäßen Mehrzweckbehälter positionierbar ist. Bevorzugterweise kann der Dufteinsatz kreisscheibenförmig bzw. flach geformt sein und einen ringförmigen Querschnitt aufweisen, welcher einen ungehinderten Zugang zu der Öffnung des Deckels ermöglicht.

Der Dufteinsatz weist dafür vorzugsweise einen mit der Öffnung des Deckels in Überdeckung bringbaren, entfernbaren Mittelbereich auf, der über eine Materialschwächung mit einem äußeren Ringbereich verbunden ist. Bei Verwendung des Dufteinsatzes im Mehrzweckbehälter kann dieser Mittelbereich einfach manuell vom übrigen Dufteinsatz getrennt werden, indem er beispielsweise herausgedrückt wird und sich dabei im Bereich der Materialschwächung ablöst. Die Öffnung bleibt somit auch bei Verwendung des Dufteinsatzes frei zugänglich. Der Dufteinsatz kann aber alternativ auch unabhängig vom Mehrzweckbehälter verwendet werden. In diesem Fall ist es vorteilhaft, den Mittelbereich im Dufteinsatz zu belassen und somit das mehr Material zur Duftabgabe zur Verfügung zu haben.

In einer bevorzugten Weiterbildung weist der Dufteinsatz eine Lasche auf, die über eine Materialschwächung mit einem äußeren Rand des Dufteinsatzes verbunden ist. Mit Hilfe dieser Lasche kann der Dufteinsatz bei einer Verwendung außerhalb des Mehrzweckbehälters beispielsweise einfach an geeigneter Stelle befestigt bzw. aufgehängt werden. Zum Einsetzen in die Aufnahmeeinheit bzw. Aufnahmevertiefung kann diese Lasche dann einfach manuell vom Dufteinsatz gelöst werden und behindert damit das Einsetzen nicht weiter.

Darüber hinaus betrifft die Erfindung eine Kartusche, welche insbesondere zum Bereitstellen von Tüchern dient. Die Kartusche ist in einem Aufnahmeraum eines Mehrzweckbehälters positionierbar, wobei eine Vielzahl von Tüchern über die Öffnung in dem Deckel des Mehrzweckbehälters entnehmbar ist. Die Kartusche kann beispielsweise zylindrisch geformt sein und Tücher bevorraten. Vorzugsweise kann die Kartusche einseitig geöffnet sein. Die geöffnete Seite der Kartusche ist in einem im Gehäuse eingesetzten Zustand zu der Öffnung des Deckels hin ausgerichtet. Durch eine derartige Kartusche ist eine besonders einfache Wiederverwendbarkeit des Mehrzweckbehälters durch den Einsatz von Nachfüllpackungen bzw. Nachfüllkartuschen möglich.

Die Kartusche ist vorzugsweise als Aluminiumhülle ausgebildet, die die als Rolle darin angeordeten Tücher umgibt. Diese Aluminiumhülle hüllt somit die Tücher ein und schützt sie gegen Austrocknung. Dabei ist natürlich eine Öffnung zum Entnehmen der Tücher in der Aluminiumhülle vorgesehen, wobei diese Öffnung beispielsweise vor Verwendung der Kartusche manuell aufgerissen werden kann. Damit kann die Kartusche längere Zeit bevorratet werden, ohne dass die Tücher austrocknen.

Weitere Merkmale, Einzelheiten und Vorteile der Erfindung ergeben sich aus dem Wortlaut der Ansprüche sowie aus der folgenden Beschreibung der Ausführungsbeispiele anhand der Zeichnungen. Es zeigen:
- Fig. 1: eine Explosionsdarstellung eines erfindungsgemäßen Mehrzweckbehälters gemäß einer Ausführungsform;
- Fig. 2: Seitenansichten des erfindungsgemäßen Mehrzweckbehälters aus Fig. 1;
- Fig. 3: eine Darstellung eines Verschlusses für einen erfindungsgemäßen Mehrzweckbehälter;
- Fig. 4: eine Schnittdarstellung zum Veranschaulichen des erfindungsgemäßen Mehrzweckbehälters im Bereich eines Deckels;
- Fig. 5: eine Draufsicht auf den erfindungsgemäßen Mehrzweckbehälters aus Fig. 1;
- Fig. 6: eine schematische Darstellung einer Dosiereinrichtung des erfindungsgemäßen Mehrzweckbehälters und
- Fig. 7: einen Dufteinsatz.

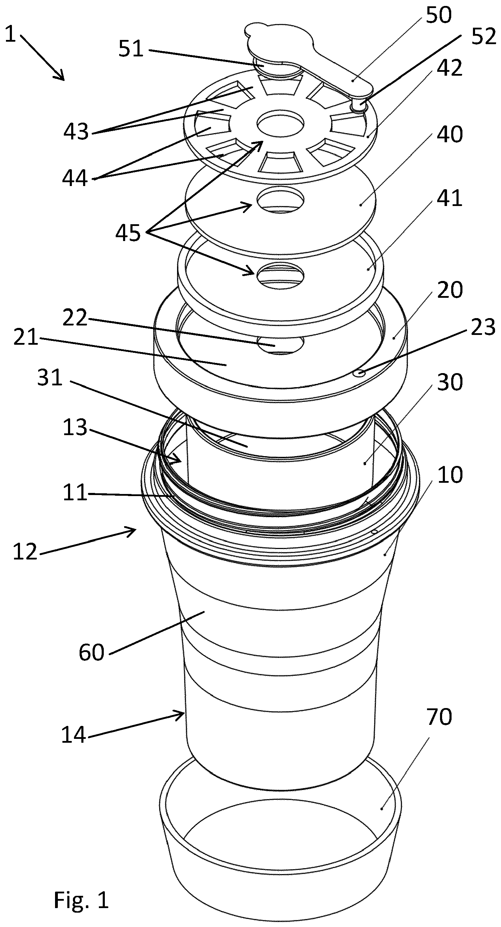

**Fig. 1** zeigt eine Explosionsdarstellung eines erfindungsgemäßen Mehrzweckbehälters 1 gemäß einer Ausführungsform. Der Mehrzweckbehälter 1 kann vorzugsweise in einem Getränkehalter eines PKW oder LKW positioniert werden und somit zusätzliche Funktionen bereitstellen.

Der Mehrzweckbehälter 1 weist ein Gehäuse 10 auf, welches becherförmig ausgeführt ist. Das Gehäuse 10 ist einseitig geöffnet und weist ein Gewinde 11 auf. Das Gewinde 11 ist an einem aufgeweiteten Abschnitt 12 des Gehäuses 10 angeordnet. Das Gehäuse 10 kann aus Kunststoff, Bambus, Holz, Glas und dergleichen hergestellt sein.

Des Weiteren kann in dem Gehäuse 10 ein Aufnahmeraum 13 ausgebildet werden. Hierzu weist der Mehrzweckbehälter 1 einen Deckel 20 auf, welcher über das Gewinde 11 mit dem Gehäuse 10 verschraubbar ist.

Der Deckel 20 besteht beispielsweise aus einem Kunststoff und kann das Gehäuse 10 endseitig verschließen. Der Deckel 20 weist eine Aufnahmevertiefung 21 auf. Die Aufnahmevertiefung 21 zeigt im eingebauten Zustand des Deckels 20 vom Gehäuse 10 fort. In den Deckel 20 ist eine Öffnung 22 eingebracht. Die Öffnung 22 ist zentriert bzw. mittig in dem Deckel 20 positioniert und stellt eine Verbindung zum Aufnahmeraum 13 des Gehäuses 10 her. Der Deckel 20 weist darüber hinaus eine Bohrung 23 auf, welche randseitig und parallel zu der Öffnung 22 in den Deckel 20 eingebracht ist.

In dem Aufnahmeraum 13 des Gehäuses 10 kann eine Kartusche 30 hineingesetzt werden. Die Kartusche 30 ist zylindrisch geformt und dient zum Bereitstellen von Tüchern 31, wie beispielsweise Reinigungstüchern. Hierzu ist die Kartusche 30 analog zum Gehäuse 10 endseitig derart geöffnet, dass die Tücher 31 über die Öffnung 22 des Deckels 20 entnehmbar sind.

In die Aufnahmevertiefung 21 kann ein Dufteinsatz 40 eingelegt werden. Der Dufteinsatz 40 ist gemäß dem Ausführungsbeispiel mittels einer Aufnahmeeinheit 41 in die Aufnahmevertiefung 21 hineinlegbar. Die Aufnahmeeinheit 41 besteht aus einem elastischen Material, wie beispielsweise Silikon oder Kautschuk, und kann neben dem Dufteinsatz 40 auch eine Dosiereinheit 42 aufnehmen. Durch die elastischen Materialeigenschaften umgreift die Aufnahmeeinheit 41 den Dufteinsatz 40 und die Dosiereinheit 42 umfangsseitig und stellt eine formschlüssige Verbindung her. Hierdurch bilden die Dosiereinheit 42, der Dufteinsatz 40 und die Aufnahmeeinheit 41 eine zusammenhängende Komponente aus, welche als Ganzes in die Aufnahmevertiefung 21 einlegbar ist.

Insbesondere liegt die Dosiereinheit 42 auf dem Dufteinsatz 40 auf, und verdeckt diesen bereichsweise. Hierzu weist die Dosiereinheit 42 Abschnitte 43 zum Verdecken des Dufteinsatzes 40 und zwischen den Abschnitten 43 ausgebildete Luftschlitze 44 zum bereichsweisen Freilegen des Dufteinsatzes 40. Der Mehrzweckbehälter 1 ist im Wesentlichen rotationssymmetrisch ausgestaltet. Daher ist auch die Aufnahmeeinheit 41, der Dufteinsatz 40 und die Dosiereinheit 42 rotationssymmetrisch ausgebildet. Hierdurch kann die Dosiereinheit 42 relativ zum Dufteinsatz 40 verdreht werden.

Zum Ermöglichen eines Zugangs zur Öffnung 22 des Deckels 20 ist in dem Dufteinsatz 40, der Aufnahmeeinheit 41 und der Dosiereinheit 42 jeweils eine zentrierte Aussparung 45 vorgesehen, um ein Verdecken der Öffnung 22 zu verhindern. Vorzugsweise sind die zentrierten Aussparungen 45 kongruent mit der Öffnung 22.

Werden feuchte Tücher 31 verwendet, kann ein optionaler Verschluss 50 vorgesehen sein. Der Verschluss 50 weist einen Spundabschnitt 51 auf, welcher durch die Aussparungen 45 in die Öffnung 22 hineinsetzbar ist. Mit dem Spundabschnitt 51 ist ein Halteabschnitt 52 verbunden. Der Halteabschnitt 52 kann in die Bohrung 23 des Deckels 20 hineingesteckt werden, um ein unbeabsichtigtes Verlieren des Verschlusses 50 zu verhindern. Vorzugsweise kann der Verschluss 50 aus einem elastischen Material ausgebildet sein.

Für den Vertrieb des Mehrzweckbehälters 1 kann darüber hinaus ein Aufkleber 60 vorgesehen sein, welcher an einer Mantelfläche 14 des Gehäuses 10 anbringbar ist. Der Aufkleber 60 kann beispielsweise für Werbezwecke oder als Dekoration eingesetzt werden.

Bodenseitig ist an das Gehäuse 10 eine Adapterhülse 70 anbringbar, durch welche der Mehrzweckbehälter 1 an Getränkehalter mit größeren Durchmessern anpassbar ist. Die Adapterhülse 70 ist als ein elastischer Überzug ausgeführt.

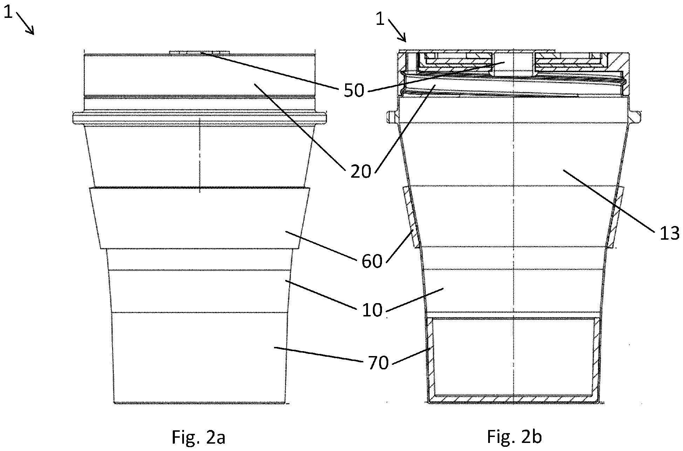

In **Fig. 2a** und **Fig. 2b** sind Seitenansichten des erfindungsgemäßen Mehrzweckbehälters 1 dargestellt. Insbesondere wird in Fig. 2b ein Querschnitt durch den Mehrzweckbehälter 1 gezeigt, welcher den Aufbau des Mehrzweckbehälters 1 im montierten Zustand veranschaulicht.

Hierbei ist der Verschluss 50 in die Öffnung 22 des Deckels 20 eingesetzt, um den Aufnahmeraum 13 des Gehäuses 10 luftdicht zu verschließen. Der Übersicht halber ist die Kartusche 30 nicht dargestellt.

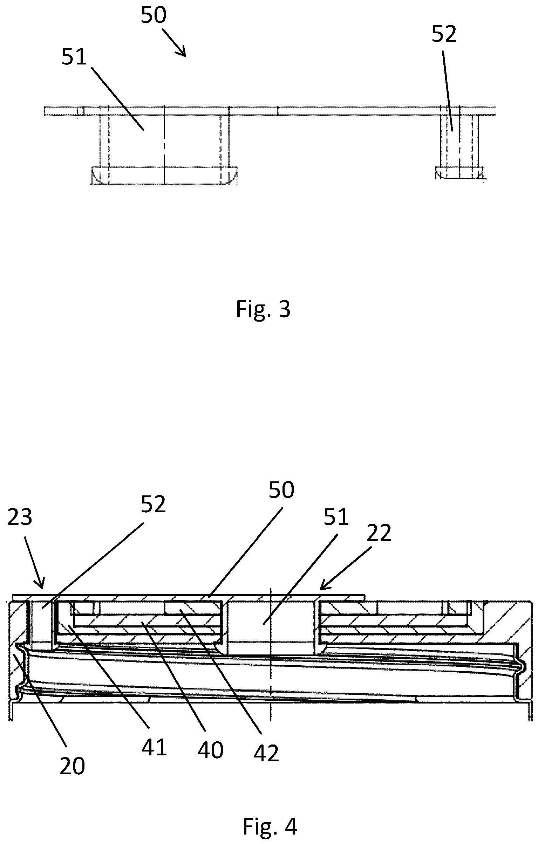

**Fig. 3** zeigt eine Darstellung eines Verschlusses 50 für den erfindungsgemäßen Mehrzweckbehälter 1. Insbesondere wird der Verschluss 50 in einer Seitenansicht dargestellt, in welcher der Spundabschnitt 51 und der vom Spundabschnitt 51 beabstandete Halteabschnitt 52 des Verschlusses 50 veranschaulicht sind.

In **Fig. 4** ist eine Schnittdarstellung zum Veranschaulichen des erfindungsgemäßen Mehrzweckbehälters 1 im Bereich des Deckels 20 gezeigt. Der Verschluss 50 ist hierbei ebenfalls in den Deckel 20 hineingesetzt, um die Öffnung 22 zu verschließen. Der Spundabschnitt 51 des Verschlusses 50 füllt die Öffnung 22 luftdicht aus. Der Halteabschnitt 52 ist in der randseitigen Bohrung 23 eingebracht.

Der Dufteinsatz 40, die Aufnahmeeinheit 41 und die Dosiereinheit 42 sind in der Aufnahmevertiefung 21 des Deckels 20 hineingesetzt und schließen bündig mit dem Deckel 20 ab.

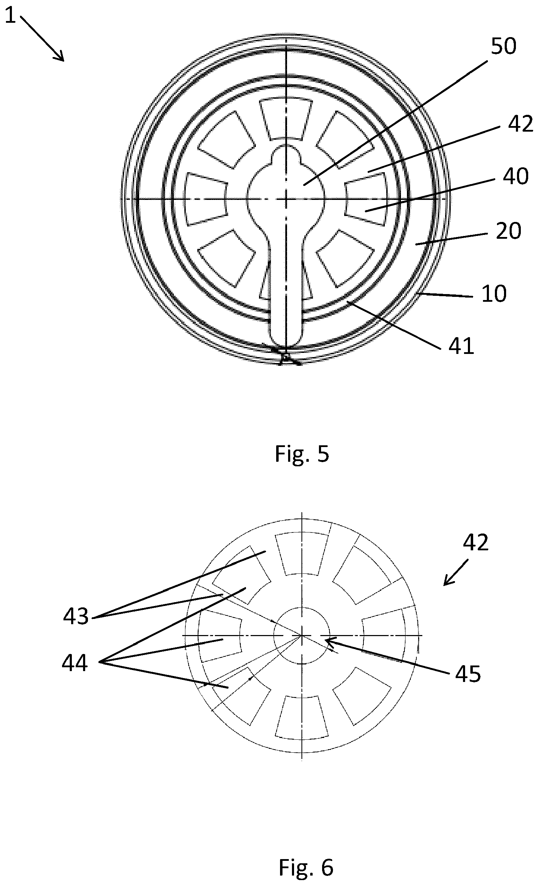

**Fig. 5** veranschaulicht in einer Draufsicht den erfindungsgemäßen Mehrzweckbehälter 1 aus Fig. 1. Es wird insbesondere die Form der Dosiereinheit 42 verdeutlicht, welche auch in der **Fig. 6** separat dargestellt ist. Die in die Aufnahmevertiefung 21 hineingesetzte Aufnahmeeinheit 41 füllt den Zwischenraum zwischen der Dosiereinheit 42 und dem Deckel 20 radial bzw. umfangsseitig aus und arretiert somit die Dosiereinheit 42 in der Aufnahmevertiefung 21 des Deckels 20.

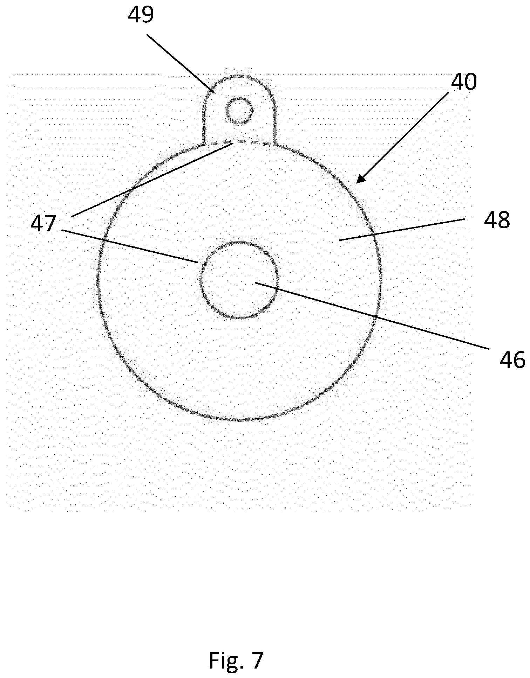

Fig. 7 zeigt nun den Dufteinsatz 40 vor Einsetzen in die Aufnahmeeinheit des Mehrzweckbehälters. Der Dufteinsatz 40 weist einen mit der Öffnung 22 des Deckels in Überdeckung bringbaren, entfernbaren Mittelbereich 46 auf. Der Mittelbereich 46 ist dabei zentrisch innerhalb des Dufteinsatzes 40 ausgebildet und weist eine Kreisform auf. Mit einem äußeren Ringbereich 48 des Dufteinsatzes 40 ist er über eine Materialschwächung 47 verbunden ist. Diese Materialschwächung 47 ist beispielsweise als Stanzung ausgeführt und ermöglicht ein einfaches manuelles Herausdrücken des Mittelbereichs 46 aus dem Dufteinsatz 40.

Der Dufteinsatz 40 weist ferner eine ebenfalls über eine Materialschwächung 47 verbundene Lasche 49 auf, die sich in einer Radialrichtung erstreckt. Aufgrund der Materialschwächung 47 kann die Lasche 49 problemlos manuell vom übrigen Dufteinsatz getrennt werden.

Durch Entfernen des Mittelbereichs 46 sowie der Lasche 49 kann der Dufteinsatz 40 passgenau in die Aufnahmeeinheit 41 eingesetzt werden, ohne dann die Öffnung 22 im Deckel 20 des Mehrzweckbehälters 1 zu überdecken. Vor Entfernen des Mittelbereichs 46 und der Lasche 49 kann der Dufteinsatz aber unabhängig vom Mehrzweckbehälter verwendet werden, wobei durch die Lasche 49 eine einfache Befestigung des Dufteinsatzes 40 ermöglicht wird und durch den Mittelbereich 46 ein zusätzliches Duftreservoir bereit gestellt wird. Der Dufteinsatz ist somit universell einsetzbar.

Die Erfindung ist nicht auf eine der vorbeschriebenen Ausführungsformen beschränkt, sondern in vielfältiger Weise abwandelbar. Beispielsweise kann der Dufteinsatz 40 ohne eine Dosiereinheit 42 in der Aufnahmevertiefung 21 anordbar sein. Des Weiteren kann die Form des Mehrzweckbehälters 1 von der dargestellten Form abweichen, wobei auch die Öffnung 22 asymmetrisch in den Deckel 20 einbringbar ist. Darüber hinaus kann der Dufteinsatz 40 in Form eines Streifens oder beispielsweise eines Ringabschnitts ausgeführt sein.

Ferner kann vorgesehen sein, den Mehrzweckbehälter mit einem oder mehren Sensoren auszustatten. Diese Sensoren können dabei rein optisch beispielsweise einen Füllstand an Tüchern anzeigen, aber auch auf Grundlage einer chemischen Reaktion Aufschluss über einen Feuchtigkeitsgehalt der Tücher oder des Dufteinsatzes geben. Ein derartiger Sensor kommt dabei ohne elektrische Energie aus.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

### Bezugszeichenliste

- 1: Mehrzweckbehälter

- 10: Gehäuse
- 11: Gewinde des Gehäuses
- 12: aufgeweiteter Abschnitt des Gehäuses
- 13: Aufnahmeraum
- 14: Mantelfläche des Gehäuses

- 20: Deckel
- 21: Aufnahmevertiefung
- 22: Öffnung
- 23: Bohrung

- 30: Kartusche
- 31: Tücher

- 40: Dufteinsatz
- 41: Aufnahmeeinheit
- 42: Dosiereinheit
- 43: Abschnitte der Dosiereinheit
- 44: Luftschlitze der Dosiereinheit
- 45: zentrierte Aussparung
- 46: Mittelbereich
- 47: Materialschwächung
- 48: äußerer Ringbereich
- 49: Lasche

- 50: Verschluss
- 51: Spundabschnitt
- 52: Halteabschnitt

- 60: Aufkleber
- 70: Adapterhülse

## Patentansprüche

1. Mehrzweckbehälter (1) mit einer Lufterfrischungsfunktion zum Aufnehmen von Tüchern (31), insbesondere für den Einsatz in einem Getränkehalter eines Fahrzeugs, aufweisend ein Gehäuse (10), einen Deckel (20) zum Verschließen des Gehäuses (10) und zum Ausbilden eines Aufnahmeraums (13) in dem Gehäuse (10) und aufweisend einen Dufteinsatz (40), wobei der Deckel (20) eine Öffnung (22) zum Entnehmen von Tüchern (31) aus dem Aufnahmeraum (13) aufweist und wobei der Deckel (20) eine Aufnahmevertiefung (21) zum Aufnehmen des Dufteinsatzes (40) aufweist.

2. Mehrzweckbehälter nach Anspruch 1, wobei der Aufnahmeraum (13) des Gehäuses (10) dazu eingerichtet ist, Tücher (31) aufzunehmen, wobei die Tücher (31) in einer Kartusche (30) oder direkt in dem Aufnahmeraum (13) einsetzbar sind.

3. Mehrzweckbehälter nach Anspruch 1 oder 2, wobei der Dufteinsatz (40) in eine Aufnahmeeinheit (41) einlegbar ist, wobei die Aufnahmeeinheit (41) mit dem eingelegten Dufteinsatz (40) in der Aufnahmevertiefung (21) des Deckels (20) platzierbar ist.

4. Mehrzweckbehälter nach einem der Ansprüche 1 bis 3, wobei der Mehrzweckbehälter (1) eine Dosiereinheit (42) aufweist, welche den Dufteinsatz (40) zumindest bereichsweise verdeckt, wobei die Dosiereinheit (42) relativ zum Dufteinsatz (40) verschiebbar oder drehbar ausgestaltet ist.

5. Mehrzweckbehälter nach einem der Ansprüche 2 bis 4, wobei die Dosiereinheit (42) in die Aufnahmeeinheit (41) oder in die Aufnahmevertiefung (21) des Deckels (20) über dem Dufteinsatz (40) drehbar positionierbar ist.

6. Mehrzweckbehälter nach einem der Ansprüche 1 bis 5, wobei der Mehrzweckbehälter (1) einen Verschluss (50) zum Verschließen der Öffnung (22) aufweist.

7. Mehrzweckbehälter nach einem der Ansprüche 1 bis 6, wobei die in den Aufnahmeraum (13) einsetzbaren Tücher (31) als Feuchttücher ausgestaltet sind, wobei das Gehäuse (10), der Deckel (20) und/oder der Verschluss (50) einen Feuchtigkeitssensor und/oder eine Feuchtigkeitsanzeige zum Anzeigen eines Feuchtigkeitsgehalts der Tücher (31) im Aufnahmeraum (13) aufweisen.

8. Mehrzweckbehälter nach einem der Ansprüche 1 bis 7, wobei die Dosiereinheit (42) einstellbare Luftschlitze (44) zum Herstellen oder zum Verändern eines Luftkontakts zum Dufteinsatz (40) aufweist.

9. Mehrzweckbehälter nach einem der Ansprüche 1 bis 8, wobei der Deckel (20) über eine Schraubverbindung oder eine Steckverbindung mit dem Gehäuse (10) verbindbar ist.

10. Mehrzweckbehälter nach einem der Ansprüche 1 bis 9, wobei die Öffnung (22) zum Aufnahmeraum (13) zentriert ausgerichtet ist und sich durch den Deckel (20), die Aufnahmeeinheit (41), den Dufteinsatz (40) und durch die Dosiereinheit (42) erstreckt.

11. Mehrzweckbehälter nach einem der Ansprüche 1 bis 10, wobei die Dosiereinheit (42) und/oder die Aufnahmeeinheit (41) und/oder der Dufteinsatz (40) über Befestigungsclips oder über Rastverbindung ortsfest in der Aufnahmevertiefung (21) des Deckels (20) platzierbar sind.

12. Dufteinsatz (40), wobei der Dufteinsatz (40) in einem Mehrzweckbehälter (1) gemäß einem der vorhergehenden Ansprüche positionierbar ist.

13. Dufteinsatz nach Anspruch 12, **dadurch gekennzeichnet, dass** er ein mit der Öffnung (22) des Deckels in Überdeckung bringbaren, entfernbaren Mittelbereich (46) aufweist, der über eine Materialschwächung (47) mit einem äußeren Ringbereich (48) verbunden ist.

14. Kartusche (30), insbesondere zum Bereitstellen von Tüchern (31), wobei die Kartusche (30) in einem Aufnahmeraum (13) eines Mehrzweckbehälters (1) positionierbar ist und wobei die Tücher (31) über eine Öffnung (22) in einem Deckel (20) des Mehrzweckbehälters (1) entnehmbar sind.
